(19) **Europäisches Patentamt / European Patent Office / Office européen des brevets**

(11) **EP 3 969 624 B1**

(12) # EUROPEAN PATENT SPECIFICATION

(45) Date of publication and mention of the grant of the patent:
**12.06.2024 Bulletin 2024/24**

(21) Application number: **21714384.1**

(22) Date of filing: **08.03.2021**

(51) International Patent Classification (IPC):
**C13K 13/00** *(2006.01)*

(52) Cooperative Patent Classification (CPC):
**C13K 13/00; A23L 33/20**

(86) International application number:
**PCT/IB2021/051898**

(87) International publication number:
**WO 2021/181239 (16.09.2021 Gazette 2021/37)**

(54) **A PROCESS FOR PRODUCING XYLOOLIGOSACCHARIDES (XOS) FROM EUCALYPTUS GLOBULUS KRAFT PULP**

VERFAHREN ZUR HERSTELLUNG VON XYLOOLIGOSACCHARIDEN (XOS) AUS EUKALYPTUS-GLOBULUS-KRAFT-ZELLSTOFF

PROCÉDÉ DE PRODUCTION DE XYLOOLIGOSACCHARIDES (XOS) À PARTIR DE PÂTE KRAFT D'EUCALYPTUS GLOBULUS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.03.2020 PT 2020116165**

(43) Date of publication of application:
**23.03.2022 Bulletin 2022/12**

(73) Proprietors:
• **RAIZ - Instituto De Investigação Da Floresta E Papel**
3800-783 Eixo, Aveiro (PT)
• **Instituto Superior Técnico**
1049-001 Lisboa (PT)

(72) Inventors:
• **DE FIGUEIREDO BRITES ALVES, Ana Maria**
1049-001 LISBOA (PT)
• **DOS SANTOS SERRANO, Maria De Lurdes**
1049-001 LISBOA (PT)
• **ANTUNES HENRIQUES, Patrícia Isabel**
1049-001 LISBOA (PT)
• **MENDES DE SOUSA, Antonio Paulo**
3800-783 EIXO (PT)

(56) References cited:
**ES-A1- 2 346 124     US-A1- 2013 296 545**

• SILVA FELIPE F ET AL: "Isolation and purification of concentrated and non-concentrated hemicellulose alkaline extracts", SEPARATION AND PURIFICATION TECHNOLOGY, vol. 173, 20 September 2016 (2016-09-20), pages 233-239, XP029803241, ISSN: 1383-5866, DOI: 10.1016/J.SEPPUR.2016.09.033
• BANERJEE SHIVALI ET AL: "Hemicellulose based biorefinery from pineapple peel waste: Xylan extraction and its conversion into xylooligosaccharides", FOOD AND BIOPRODUCTS PROCESSING, INSTITUTION OF CHEMICAL ENGINEERS, RUGBY, GB, vol. 117, 25 June 2019 (2019-06-25), pages 38-50, XP085805689, ISSN: 0960-3085, DOI: 10.1016/J.FBP.2019.06.012 [retrieved on 2019-06-25]
• CHEN XUE ET AL: "Evaluating the production of monosaccharides and xylooligosaccharides from the pre-hydrolysis liquor of kraft pulping process by acid and enzymatic hydrolysis", INDUSTRIAL CROPS AND PRODUCTS, ELSEVIER, NL, vol. 124, 19 September 2018 (2018-09-19), pages 906-911, XP085484724, ISSN: 0926-6690, DOI: 10.1016/J.INDCROP.2018.08.071

EP 3 969 624 B1

- ZHANG HONGYU ET AL: "Co-production of functional xylooligosaccharides and fermentable sugars from corncob with effective acetic acid prehydrolysis", BIORESOURCE TECHNOLOGY, ELSEVIER, AMSTERDAM, NL, vol. 234, 22 February 2017 (2017-02-22), - 22 February 2017 (2017-02-22), pages 343-349, XP029964242, ISSN: 0960-8524, DOI: 10.1016/J.BIORTECH.2017.02.094
- MARQUES ANA ISABEL ET AL: "Isolation of xylans from bleached Eucalyptuskraftpulp by antisolvents precipitation", CELLULOSE, SPRINGER NETHERLANDS, NETHERLANDS, vol. 26, no. 3, 23 July 2018 (2018-07-23), pages 1977-1992, XP036721482, ISSN: 0969-0239, DOI: 10.1007/S10570-018-1941-X [retrieved on 2018-07-23]

**Description**

Technical Field

The present invention relates to a process for producing xylooligosaccharides (XOS) involving a direct acid hydrolysis of an alkaline Kraft filtrate of *Eucalyptus globulus,* at moderate experimental conditions. The invention is defined by the appended claims.

Background Art

In numerous scientific publications, several authors have associated to prebiotics, and their use as food additives, several beneficial effects on physical health (Binns, 2013; Carlson et al., 2018; Enam and Mansell, 2019; Shortt et al., 2018), and even more recently, regarding human mental health (Appleton, 2018; Cryan et al., 2019; Kim and Shin, 2018; Kowalski and Mulak, 2019). Effects on animal health condition have also been reported (Ohbuchi et al., 2010). Another significant factor reflecting the recent interest in these food additives is the enormous size of the prebiotic market, with a compound annual growth rate of 10.4% expected over the next decade, with this market expected to reach 7.37 billion US dollars by 2023 (Markets and Markets, 2018).

Two main groups constitute the most studied and used prebiotics: inulin-type fructans (inulin, oligofructose and fructooligosaccharides (FOS)) and galactans (galactooligosaccharides (GOS)) (Gibson et al., 2017; Roberfroid et al. 2010).

However, the growing demand for prebiotics has led both academia and industry to look for new potential candidates. Of these, xylooligosaccharides (XOS), a type of emerging prebiotics obtained from xylans (Akpinar et al., 2010; Cao et al., 2018; Mafei et al., 2019; Mazlan et al., 2019; Wen et al., 2019), stand out, already being certified products as Generally Recognized as Safe (GRAS), that is, generally recognized as safe by the US Food and Drug Administration, FDA, (FDA, 2019, 2013), as well as also considered safe as a new food, according to regulation (EU) 2015/2283 of the European Food Safety Authority (EFSA) (EFSA, 2018).

Important benefits of XOS over galactooligosaccharides (GOS) and fructooligosaccharides (FOS) have already been listed. XOS have, in general, good organoleptic properties, as well as a greater stability to heat and pH, properties of high relevance for the food industries (Mano et al., 2018). Of the various existing XOS, those with a lower degree of polymerization, such as xylobiose (X2) and xylotriose (X3), are reported as having the greatest prebiotic effect, being the most recommended for food and nutraceutical applications (Moura et al. , 2007; Rajagopalan et al., 2017; Singh et al., 2018). Xylans, the type of hemicelluloses most abundantly available in lignocellulosic materials, can be extracted from a wide variety of lignocellulosic biomass, including various types of wood. The main polymeric components of lignocellulosic biomass are cellulose (35-50%), lignin (5-30%) and hemicelluloses (20-35%), with xylans accounting for 95% of the latter (Cao et al., 2018). Hemicelluloses can consist of the same monomer or of different monosaccharides, and can have different chains and branches sizes. According to the monomeric component, hemicelluloses can be subdivided into xylans, mannans, glucans and galactans (Evtuguin et al., 2017). Xylans are polymers of the monomeric units pentoses, that is, they are classified as pentosans. These pentosans, when hydrolyzed, essentially result into xyloses.

The production of XOS from lignocellulosic biomass can be considered a difficult task. A pre-treatment is generally recommended for the separation of the different components constituting the lignocellulosic biomass, thus making hemicelluloses, and therefore xylans, more available for their conversion into XOS (Taha et al., 2016). The use of hot water (Batista et al., 2019), steam-induced explosion (Bhatia et al., 2020; Silveira et al., 2018) and chemical treatments (Quiñones et al., 2015) are the pre-treatments most commonly reported in the literature. Among the chemical treatments, the alkaline process is considered one of the most efficient in terms of recovering xylans without their degradation (Amorim et al., 2019). The conversion processes of isolated xylans into XOS are carried out mainly by self-hydrolysis, acidic or enzymatic procedures (or a mixture of the two), and the degree of polymerization of the obtained XOS and the yield of conversion depend on the nature of the raw material and on its processing (Kaprelyants et al., 2017; Wang et al., 2019) .

Self-hydrolysis requires specialized equipment to withstand the high temperatures and pressures involved, also leading to the production of other secondary products (Carvalho et al., 2013; Mazlan et al., 2019; Samanta et al., 2015), in general, unwanted.

Enzymatic hydrolysis, considered to be more environmentally friendly and suitable for pharmaceutical and food applications (Amorim et al., 2019) is, however, a complex process with low reaction speeds, low yields (Wang et al., 2019) and with high costs associated with the consumption of enzymes.

Acid hydrolysis is negatively associated with the corrosion of the involved equipment and with the production of large amounts of xylose and other by-products such as furfural and hydroxymethylfurfural (HMF) (Akpinar et al., 2010; Zhang et al., 2017). However, despite these disadvantages, this method has been widely studied (Akpinar et al., 2010;

Chen et al., 2018; Hachem et al., 2017; Wang et al., 2018; Zhang et al., 2018; Zhang et al., 2017; Zhou and Xu, 2019), as a result of its high reaction speeds (Wang et al., 2019), the achievement of a wide range of degrees of polymerization, from 2 to 15 (Kaprelyants et al. 2017) and, mainly, due to the possibility of large-scale production with low associated costs. The by-products can, in turn, be removed through various purification processes, such as solvent extraction and precipitation (Moure et al., 2006; Swennen et al., 2005), activated carbon adsorption, ion chromatography processes (Qing et al., 2013) and membrane processes. In contrast to the latter, the other processes have associated a few concerns regarding food applications, environmental impact and industrial scale execution (Aachary and Prapulla, 2011) . Membrane processes have come to be considered very promising to fractionate, purify and concentrate XOS (Córdova et al., 2019), as they have several advantages such as: direct scale-up, energy efficiency and easily optimized operating conditions (Patil et al. , 2014). Ultrafiltration (UF) has been widely used for removing molecules of higher molecular weight that are not hydrolyzed (Akpinar et al., 2010; Kumar and Satyanarayana, 2015; Nabarlatz et al., 2007; Swennen et al., 2005). This can be complemented with nanofiltration to separate xylose and salts (Hua et al., 2010; Vegas et al., 2006; Yuan et al., 2004), sometimes with simultaneous diafiltration (Zhao et al., 2012).

The work of Akpinar and co-authors consists on the use of tobacco branch as a source of xylans for the production of XOS, through an acidic and enzymatic hydrolysis. Acid hydrolysis was carried out through the use of sulfuric acid with a final neutralization step with calcium carbonate (Akpinar et al., 2010). The results are characterized by low yields and by the suggestion of the need of using additional steps involving the combination of both enzymatic and acidic procedures . The same acid was used by Chen and his co-authors (Chen et al., 2018) for the production of XOS from the pre-hydrolyzed liquor of a Kraft pulp production process, requiring the use of high temperatures (120°C) and reaction times of up to two hours, in order to reduce the concentration of the used sulfuric acid. The same type of results was obtained by Wang and coauthors (Wang et al., 2018), that is, the use of sulfuric acid at considerably high temperatures as well as long reaction times.

The pulp and paper industry uses different mechanical and chemical processes for removing cellulose for papermaking. Among the available processes there is the Kraft process, or the sulfate process, which uses sodium hydroxide and sodium sulfide as reagents. The cellulose pulp resulting from this process is called Kraft pulp. The bleached Kraft pulp, produced from *Eucalyptus globulus* wood, is as rich in xylans as the original wood from where it comes from (16 to 23%) (Marques et al., 2019; Shatalov et al., 1999), consequently constituting an enormous potential for the production of a wide variety of products with high added value, including XOS. Nevertheless, it is still rarely used (Hakala et al., 2013; Monrroy et al., 2012; Ohbuchi et al., 2009).

Silva et al. 2017, discloses the study of separation processes of hemicelluloses (xylans) contained in paper pulp obtained by a kraft process. The processes of alkaline extraction of xylans from the pulp, ultrafiltration and precipitation by solvents were there discussed. There is no demonstration and exemplification of the conversion of these xylans to another product, like xylooligosaccharides, either by acid hydrolysis or otherwise. The mentioned nitric acid precipitation relates to the processes of precipitation by solvents for the extraction and isolation of xylans.

Banerjee et al. 2019, describes the production of hemicelluloses from pineapple peels. The document describes a hydrolysis of pineapple peel with dilute nitric acid to produce a xylose-rich liquor. As a second step, this liquor is then subjected to enzymatic hydrolysis to produce xylooligosaccharides.

Chen et al. 2018, describes the use of sulfuric acid for the production of XOS from the pre-hydrolyzed liquor of a kraft pulping process, requiring the use of high temperatures (120 °C) and reaction times of up to two hours, in order to reduce the concentration of sulfuric acid used.

US20130296545A1 describes a process for producing dissolving cellulose. It also describes production of xylooligosaccharides by hydrothermolysis or by enzymatic hydrolysis.

ES2346124A1 describes purification processes of xylooligosaccharides solutions that involve different processes such as diafiltration, ultrafiltration, nanofiltration, adsorption and the use of resins and dehydration.

EP0988402A1 describes the process of producing xylose from a hardwood pulp, in which an enzymatic treatment is used to improve the quality of the pulp as a source of xylans and thus also improving the yield obtained in xylose, namely through the use of a xylanase enzyme. The document CN102277761B refers to the production of oligosaccharides and paper pulp, from straw residues from agricultural farming. This method involves an alkaline pre-extraction, followed by membrane processes for concentration of the xylan solution and preparation of the xylooligosaccharide solution through enzymatic reactions, vacuum concentration, precipitation lyophilization and centrifugation.

EP2748341B1 describes a process of acid hydrolysis of lignocellulosic biomass, which allows obtaining few degradation products. However, this process takes place at high temperatures, up to 200°C, and involves the addition of a salt.

Analyzing the previous techniques it is shown the nonexistence of a simple, single step and direct process, with moderate operating conditions, without the need for special equipment, which allows the production of XOS in favorable quantities, at an industrial level, through a raw material easily accessible from production processes frequently used in the production of pulp and paper. The process described in this invention is intended to remedy these shortcomings of the prior art.

## Summary of Invention

It is therefore the object of the invention a process for producing XOS from the filtrate resulting from an alkaline extraction of the *Eucalyptus globulus* Kraft pulp, according to claim 1, which involves the steps of concentrating an alkaline filtrate from a Kraft pulp of *Eucalyptus globulus* by ultrafiltration, the following acid hydrolysis of the resulting alkaline filtrate with nitric acid, at pHs between 0.5 and 1.5 and at temperatures between 70 and 100 ° C and purificating the xylooligosaccharides solution.

The XOS production process of this invention consists of a single, simple step, at moderate operating conditions, which can take place in common industrial equipment, in a short time, through an easily accessible raw material, which does not compete with food resources and is often used for the production of pulp and paper, allowing to obtain an XOS solution with a wide range of degrees of polymerization, from xylobiosis (X2) to xylohexaose (X6). In addition, unwanted degradation products, characteristic of this type of processes, such as, for example, furfural and hydroxymethylfurfural (HMF), are not found in the process disclosed. In addition, the XOS solution obtained by this process also contains xylose (X1), which can be later used, after its separation from the XOS solution obtained by the process described in this invention, per se or for the production of other high added value products, as it is with the case of furfural. Thus, the new process described here, providing a solution of XOS and xylose, without the common degradation products, allows for a later simpler separation of xylose for its future conversion into new products with high added value.

The acid hydrolysis of the invention described in this document occurs at pHs between 0.5 and 1.5 and at temperatures between 70 and 100°C. Preferably, the reaction takes place at a pH of 0.5 to 1 and at temperatures between 85 and 100°C. In an even more preferred embodiment of the invention, the acid hydrolysis reaction takes place at a pH of 0.5 and at a temperature of 100°C.

More than 30% by mass of XOS/xylans, with a high and desired formation of X2 and X3 (> 40%) and without sugar degradation by-products, are obtained in 30 minutes at 100°C and pH 0.5.

In addition to the single acid hydrolysis step of the alkaline extraction filtrate of the bleached Kraft pulp, this invention also considers preferential previous and subsequent steps, which allow a set and a sequence of unit operations with additional and complementary advantages to the single step of acid hydrolysis with nitric acid described in this invention. Namely, a previous concentration, by ultrafiltration, of the alkaline filtrate, allows a decrease in the consumption of NaOH and of $HNO_3$ at the alkaline pretreatment and at the acid hydrolysis, respectively. The possible subsequent purification of the XOS solution by nanofiltration shows low values of rejections to the nitric acid salts, since these monovalent salts (nitrates) and other monovalent ions in solution permeate together with the other contaminants, with the XOS mostly remaining in the final concentrate. This nanofiltration step can be considered as a pre-purification step complemented by other purification techniques, such as solvent precipitation.

The use of the filtrate from the alkaline extraction of bleached Kraft pulp from *Eucalyptus globulus,* as a substrate for acid hydrolysis, brings several advantages to the process. This material does not contain lignin, which, when existing, represents an added difficulty for the production of XOS. In addition, it also has the advantage of avoiding the entire process of isolation, precipitation and purification of xylans and, therefore, their associated costs.

## Brief Description of Drawings

Figure 1. Yield in XOS as a function of time for pH 0.5 (A) and 1.0 (B) at different temperatures.
Figure 2. Degrees of polymerization (DP's) distribution from X1 to X6 during the reactions of pH 1.0 and 100°C (A); pH 0.5 and 100°C (B) and 95°C (C) .
Figure 3. Xylose yield as a function of time for pH 0.5 (A) and 1.0 (B) at different temperatures.
Figure 4. Yield in XOS (A) and xylose (B) as a function of time at 100°C and different pH values.
Figure 5. Yield in XOS (A) and xylose (B) as a function of time at 100°C, pH 0.5 and different xylans concentrations.

## Detailed Description and Description of Preferred Embodiments

For the description of this invention, different analytical methods are listed below:

• Humidity and ashes

The methodologies mentioned in the standards TAPPI T 412 om-11, 2011 and TAPPI T211 om-02, 2002, were used in order to quantify the humidity and the ash content of the pulps before and after alkaline extraction, respectively.

• Pentosans content

An acid hydrolysis for carbohydrate analysis (Selvendran et al., 1979; Shatalov et al., 1999) is performed for the quantification of xylans in the pulp (before and after extraction) and in the final precipitate of the acid hydrolysis, through the content in pentosans. Carbohydrates are subjected to a treatment with 400 μL of 72% sulfuric acid (w / w) at 25°C for 3 hours, followed by dilution with 4.4 mL of distilled water at 100°C for 2.5 hours. The amounts of xylose in the resulting samples are determined by analysis with High Performance Liquid Chromatography (HPLC). By multiplying the quantities of xylose in the samples by a constant factor of 0.88, the corresponding pentosans content is obtained.

• HPLC analysis

High Performance Liquid Chromatography (HPLC) for quantification of XOS, neutral sugars and degradation products is used to analyze acid hydrolysis samples, passing them through a 0.2 um syringe filter. The XOS were eluted with milli-Q water at 70°C and 0.2 mL / min using an Aminex HPX 42A column from Biorad® and a refractive index (IR) detector model 133 Gilson. XOS are quantified only in terms of xylobiose to xylohexaose. The remaining products are eluted with 5 mM sulfuric acid at 50°C and 0.2 mL / min, using a Biorad® Aminex HPX 87H column and a model 2142 LKB Bromma RI detector. The detection limit is $\geq 0.05$ g / L.

Bearing in mind that the amount of xylans obtained in the filtrate differs for each experimental test, after analysis by HPLC, the yields in XOS and xylose were normalized by the presented equations:

$$XOS\ yield\ (\%) = \frac{m_{X2+X3+X4+X5+X6}(g)}{m_{xylan\ in\ the\ alkaline\ filtrate}(g)} \times 100 \qquad \text{Equation 1}$$

$$Xylose\ yield\ (\%) = \frac{m_{Xylose}(g)}{m_{xylan\ in\ the\ alkaline\ filtrate}(g)} \times 100 \qquad \text{Equation 2}$$

Total Organic Carbon (TOC)

For the concentration of the alkaline filtrate by ultrafiltration, the membrane rejection (R) was calculated by TOC measurements, comparing the concentration of the feed and of the permeate as followed:

$$R = 1 - \frac{c_p}{c_b} \qquad \text{Equation 3}$$

where $C_p$ and $C_b$ (mg Carbon/ L) are the concentration of the permeate and feed, respectively.

Examples

Bleached Kraft pulps (two lots) of *Eucalyptus globulus* were analyzed for their pentosans and ash content, the results of which are shown in Table 1.

*Table 1 - Characteristics of the bleached Eucalyptus pulps.*

|  | **Pentosans** | **Ashes** |
|---|---|---|
| Pulp 1 | 20.9 ± 0.2 | 0.28 ± 0.01 |
| Pulp 2 | 21.4 ± 0.4 | 0.25 ± 0.01 |

Standard XOS solutions with different degrees of polymerization (DP), xylobiose (X2), xylotriose (X3), xylotetraose (X4), xylopentaose (X5) and xylohexaose (X6) were purchased through Megazyme, Ireland. The remaining chemical reagents, including xylose (X1)> 99%, nitric acid (HNO3) 65% and sodium hydroxide (NaOH) 99%, were purchased from Sigma-Aldrich Company, USA.

Alfa Laval GR90PP membranes, with a molecular weight limit of 5 kDa, are used in ultrafiltration (UF) procedures.

The pulp is initially suspended in a 10% (w / w) NaOH solution, with a consistency of 5% (g of paste / g of total solution), and stirred for 1 hour at 25°C. The suspension is then subjected to vacuum filtration, yielding an exhausted pulp and an alkaline filtrate, containing xylans. The exhausted pulp is washed, dried at 60°C and then analyzed for humidity and pentosans and ashes content. The exhausted pulp can later be converted into dissolving pulp, a secondary product with high added value.

By comparing the initial and final amount of pentosans in the pulp, yields of xylan extraction are calculated in the range of 76 to 83%. The amount of xylans calculated is used to normalize the results of the acid hydrolysis, as described in Equations 1 and 2.

Batch mode ultrafiltration with a constant transmembrane pressure of 5 bar is used to concentrate the alkaline filtrate. Membrane rejection and concentration factors (FC) are determined by calculating the total organic carbon (TOC).

The alkaline filtrate is acidified with nitric acid to pH values between 0.5 and 1.5, and the resulting suspension is heated and stirred (100 rpm) at a constant temperature (Table 2) for up to 5 hours in an oil jacketed reactor.

Temperature control is carried out by an electronic contact thermometer VT-5 from VWR ($\pm$ 0.2°C). Table 2 presents the experimental conditions considered regarding the pH and the reaction temperature. The samples are collected every 30 minutes, neutralized with a NaOH solution and analyzed by HPLC (High Performance Liquid Chromatography) for quantifying XOS and degradation products.

After the end of the reaction, the suspension is centrifuged to obtain a solid precipitate and a supernatant. The supernatant is then analyzed by HPLC and the precipitate is dried at 60°C and then analyzed for its pentosans content.

Table 1 - Acid hydrolysis experimental conditions.

| Acid hydrolysis experimental conditions | | | | |
|---|---|---|---|---|
| Temperature (°C) | 70 | 85 | 95 | 100 |
| pH | 0.5 | 0.5 | 0.5 | 0.5 |
| | | 1.0 | 1.0 | 1.0 |
| | | | | 1.5 |

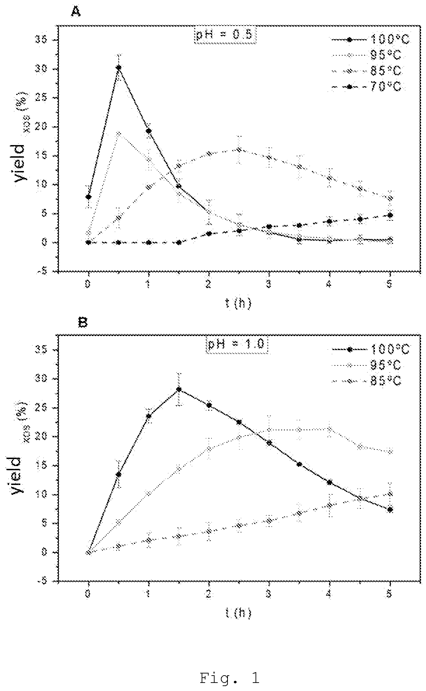

Figure 1 depicts the comparison of the yields in XOS for different temperatures and for fixed pH values of 0.5 (A) and 1.0 (B). The results show a maximum yield for the temperature of 100°C: 30.2% (30 min) for pH 0.5 and 28.2% (1h30) for pH 1.0. For a temperature of 95°C, the yield values are 18.8% (pH 0.5; 30 min) and 21.4% (pH 1.0; 4h). For temperatures of 85°C and 95°C and pH 0.5, similar yields were obtained, around 16.0%, requiring two additional hours for the case of 85°C.

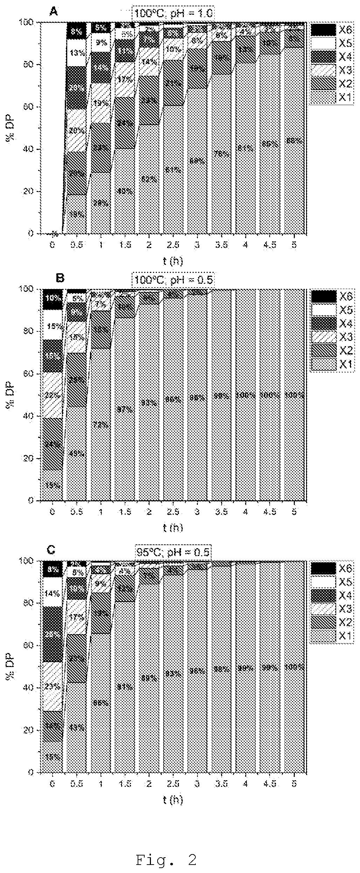

For all temperatures, at pH 1.0, a wide range of degrees of polymerization, DP's, of XOS, from X2-X6, is obtained during the reaction of five hours, always with a predominance of X2 and X3. The amount of XOS with higher polymeric degrees decreases with time, as can be seen for the temperature of 100°C in Figure 2-A, where the distribution of DP's from X1 to X6 is depicted. After 3 hours, the total amounts of X4, X5 and X6 becomes residual (<4%).

For pH 0.5, the range from X2 to X6 was obtained during the whole reaction for the lowest temperatures, of 70 and 85°C. At 100°C, Figure 2-B, after reaching the maximum yield, at 30 minutes, there was a rapid disappearance of the highest DP's of the XOS. After 1 hour of reaction, there was only xylobiose to xylopentaose, the latter disappearing after another 30 minutes of reaction. After 3 hours, only X2 remained, which, however, disappeared completely after 4 hours. Compared with a slightly lower temperature of 95°C, in Figure 2-C, a loss of the highest DPs is also observed during the reaction, although more slowly, following the order: X6, X5, X4, X3 and X2 disappeared after 1.5 to 5 hours, respectively.

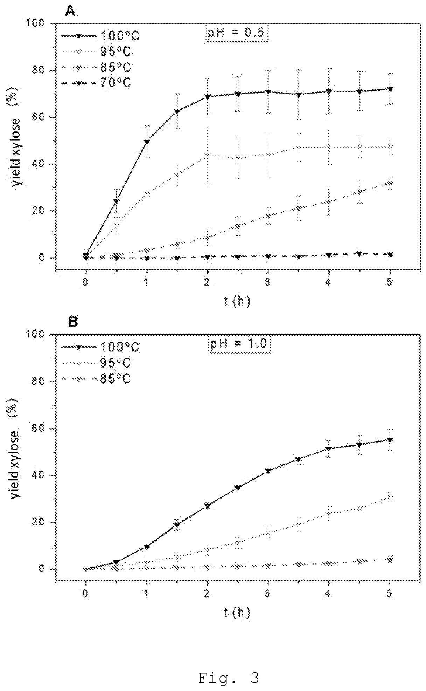

When a maximum XOS yield is reached before the 5 hours reaction (Figure 1), the yield in X1, shown in Figure 3, exceeds the yield in XOS right after that maximum, since the XOS breaks into lower polymeric grades and into xylose. Additionally, there is a stabilization of the X1 production after some time.

For a pH 0.5 (Figure 3-A) and temperatures of 95 and 100°C, the amount of X1 is constant after 2 hours, probably due to the fact that the amount of XOS available for conversion is small after this period of time. However, it is still verified, for both temperatures, that stabilization occurs for different amounts of xylose: the maximum production of X1 is greater for 100°C. Such observation is in accordance with the greater production of XOS, which, in turn, decompose into xylose.

For pH 1.0 and 100°C (Figure 3-B), there is also a tendency for the xylose yield stabilization, however much less pronounced. Like the formation of XOS, the reaction kinetics for the production of X1 is slower at lower temperatures.

The appearance of unwanted degradation products, such as, for example, furfural and HMF, has been one of the main arguments recurrently used against acid hydrolysis of lignocellulosic materials (Akpinar et al., 2010; Zhang et al., 2017). However, for all the experimental conditions of this invention, none of these secondary degradation products were detected in the HPLC analysis.

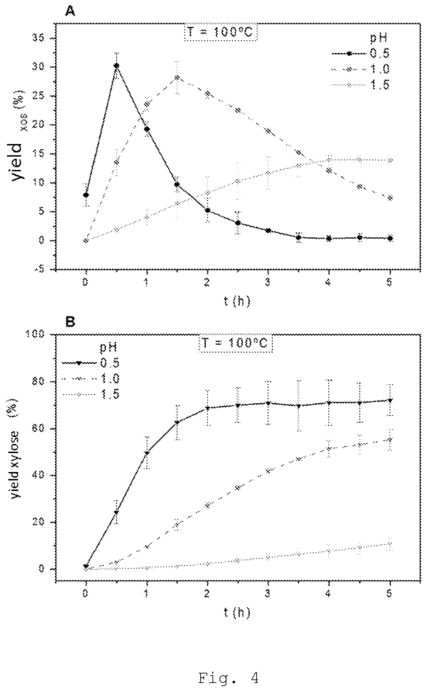

Figure 4 represents the pH influence on the production of XOS (A) and xylose (B) at 100°C. The formation of both compounds decreases as the pH increases. For pH 0.5 and 1.0, maximum XOS yields are achieved in 30 minutes and 1 hour, respectively. The formation of xylose reaches a faster stabilization at the lower pH.

Although there is a delay between the maximum yields obtained for pH 0.5 and 1.0, their values differ only by 2%. The DP's represented in Figure 2 are also similar for both pH values.

The most abundant XOS, X2 and X3, together represent 40% and 41% of the total produced, for pH 0.5 and 1.0, respectively. X4 represents 9% and 11%, X5 - X6 only 6% and 8%, and the amount of xylose is greater than 40% in both cases. The same results can be achieved in just 30 minutes, for pH 0.5. In addition, shorter reaction times are an important requirement from the point of view of an industrial application.

The XOS yields obtained are summarized in Table 3, with the respective xylose yields and times and for the same reaction time.

Table 3 - Summary of the yields in XOS with the respective times and yields in xylose.

| T (°C) | pH = 0.5 | | | pH = 1.0 | | | pH = 1.5 | | |
|---|---|---|---|---|---|---|---|---|---|
| | t (h) | XOS | X1 | t (h) | XOS | X1 | t (h) | XOS | X1 |
| 100 | 0.5 | 30.2 ± 2.2% | 24.4 ± 5.0% | 1.5 | 28.2 ± 2.8% | 19.1 ± 2.2% | 4.5 | 14.0 ± 0.6% | 9.3 ± 2.8% |
| 95 | 0.5 | 18.8 ± 0.2% | 13.9 ± 3.4% | 4.0 | 21.4 ± 1.4% | 23.6 ± 2.9% | | | |
| 85 | 2.5 | 16.0 ± 2.4% | 13.7 ± 4.1% | 5.0 | 10.1 ± 1.9% | 4.1 ± 1.3% | | | |
| 70 | 5.0 | 4.7 ± 0.9% | 1.2 ± 0.7% | | | | | | |

For the experimental conditions of this invention, with a pH of 0.5, a temperature of 100°C and a reaction time of 30 minutes, an XOS yield of 30.2% is obtained. It is also observed that the amount of xylose present in the XOS mixture is considerably high, 45% (Figure 2-B). As previously mentioned, HPLC analysis of acid hydrolysis samples did not show secondary products, such as furfural or HMF, with xylose being the only additional product to XOS, whose reported commercial purities are between 75 and 95% (Moure et al., 2006). There is an interest in removing the xylose produced by adding a purification step after acid hydrolysis. The presence of large amounts of xylose in food applications is undesirable (<12%, FDA, 2019), as it is carcinogenic and not suitable for diabetics (Córdova et al., 2019) . On the other hand, this xylose produced is a high value raw material, making it possible to convert it into different added value products, such as furfural. The fact that the process described in this invention makes it possible to obtain a solution of XOS and xylose, without the common degradation products, allows for a simple further separation of xylose for use in different processes for its conversion into new products.

Different means of purification can be used, alone or in combination with others, complementary to each other, such as extraction and precipitation with solvents, adsorption with activated carbon and processes with membranes.

When compared to more conservative technologies and with regard to sustainability and environmental factors, nanofiltration (NF) presents itself as an advantage (Córdova et al., 2019).

Akpinar et al., 2010, for the same temperature of 100°C and a reaction time of 30 minutes, only achieved a maximum yield of 13% with an acid hydrolysis of solid xylan using sulfuric acid 0.25 M. Chen et al., 2018, however, reached a yield of 30%, again with sulfuric acid (0.3%), but at 120°C and 2 hours of reaction. Wang et al., 2018 and Zhang et al., 2017, were able to obtain yields above 45%, although also with higher temperatures, of 120 and 140°C, respectively, and with sulfuric (1%) and acetic (20%) acids.

An analysis of previous scientific publications shows that most studies on acid hydrolysis of lignocellulosic material use sulfuric acid (Akpinar et al., 2010; Chen et al., 2018; Wang et al., 2018). However, the use of nitric acid has a relevant advantage when a subsequent purification of XOS by nanofiltration is envisaged. Sulfate rejection, using nanofiltration, is generally greater than 90%, while for monovalent salts of nitric acid (nitrates) the reported rejections are much less. Using nanofiltration, it is expected that nitrates and other monovalent ions in solution will permeate together with the other contaminants, while the XOS remain mainly in the concentrate. Furthermore, assuming that most of the xylose is also separated by nanofiltration, it results in a more economical process, as only a purification step would be necessary to remove salts and X1.

For some experimental conditions, the alkaline filtrate was ultrafiltered, with the main objective to examine whether the results of acid hydrolysis would be the same for an alkaline filtrate as such or for a concentrate.

For a concentration factor of 2.5, the permeate fluxes remained stable during the ultrafiltration tests, with an average value of 35 L / h.m$^2$. The membrane rejections, calculated by TOC, were 87%, which represents a good performance for this type of separation. The 13% of organic matter present in the ultrafiltration permeate is presumably of oligosaccharides that resulted from some degradation of the xylans during the alkaline extraction. With a molecular weight limit, MWCO, of 5 kDa, the GR90PP membrane would, in theory, reject all xylans (MW ≥ 20 kDa; Silva et al., 2017).

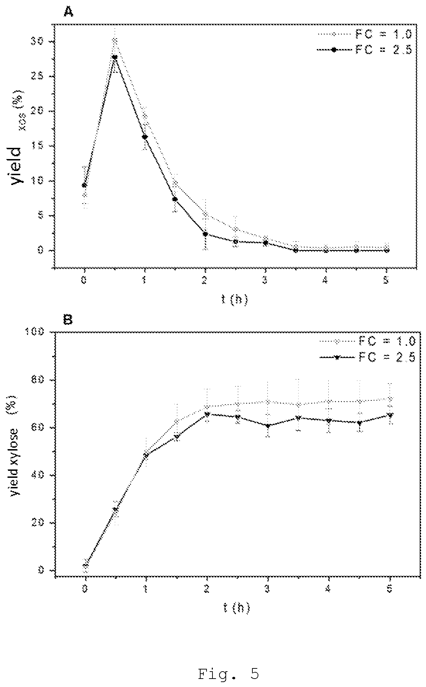

Figure 5 shows a comparison of the yields in XOS (A) and xylose (B) as a function of time, for 100°C, pH 0.5, and for the minimum and maximum concentrations considered. It is possible to observe that the yields in XOS and xylose remain very similar, even for a 2.5 times concentrated alkaline filtrate. Additionally, the distribution of DP's is identical for the maximum XOS yield (30 minutes) and, as for xylose, its amount remains above 40%. Xylobiose and xylotriose together represent 39% and the rest X4 - X6, 14%.

References

Aachary, A.A., Prapulla, S.G., 2011. Compr. Rev. Food Sci. Food Saf. 10, 2-16.

Akpinar, O., Erdogan, K., Bakir, U., Yilmaz, L., 2010. Food Sci. Technol. 43, 119-125.

Amorim, C., Silvério, S.C., Prather, K.L.J., Rodrigues, L.R., 2019. Biotechnol. Adv. 37.

Appleton, J., 2018. Integr. Med. 17, 28-32.

Batista, G., Souza, R.B.A., Pratto, B., dos Santos-Rocha, M.S.R., Cruz, A.J.G., 2019. Bioresour. Technol. 275, 321-327.

Banerjee, S., Patti, A.F., Vijayaraghavan, R., Arora, A., 2019. FOOD BIOPROD PROCESS 117, 38-50.

Bhatia, R., Winters, A., Bryant, D.N., Bosch, M., Clifton-Brown, J., Leak, D., Gallagher, J., 2020. Bioresour. Technol. 296.

Binns, N., 2013. Probiotics, prebiotics and the gut microbiota, International life sciences institute europe concise monograph series.

Cao, L., Chen, H., Tsang, D.C.W., Luo, G., Hao, S., Zhang, S., Chen, J., 2018. J. Clean. Prod. 178, 572-579.

Carlson, J.L., Erickson, J.M., Lloyd, B.B., Slavin, J.L., 2018. Curr. Dev. Nutr. 2, 1-8.

Carvalho, A.F.A., Neto, P. de O., da Silva, D.F., Pastore, G.M., 2013. Food Res. Int.

Chen, X., Cao, X., Sun, S., Yuan, T., Shi, Q., Zheng, L., Sun, R., 2018. Ind. Crops Prod. 124, 906-911.

Córdova, A., Astudillo, C., Illanes, A., 2019. Membrane Technology. pp. 113-153.

Cryan, J.F., O'riordan, K.J., Cowan, C.S.M., Sandhu, K. V et al., 2019. Physiol. Rev. 99, 1877-2013.

EFSA, P. on D.P.N. and A. (NDA), 2018. Safety of xylooligosaccharides (XOS) as a novel food pursuant to Regulation (EU) 2015/2283. EFSA J.

Enam, F., Mansell, T.J., 2019. J. Ind. Microbiol. Biotechnol. 46, 1445-1459.

FDA, 2019. GRAS Notice No. GRN 000816.

FDA, 2013. GRAS Notice (GRN) No. 458.

Evtuguin, D.V., Pascoal Neto, C. (2007). Recent advances in Eucalyptus wood chemistry: structural features through the prism of technological response.

Gibson, G.R., Hutkins, R., Sanders, M.E., Prescott, S.L., Reimer, R.A., Salminen, S.J., Scott, K., Stanton, C., Swanson, K.S., Cani, P.D., Verbeke, K., Reid, G., 2017. Nat. Rev. Gastroenterol. Hepatol. 14:491.

Hachem, K., Faugeron-Girard, C., Kaid-Harche, M., Gloaguen, V., 2017. Cogent Chem. 3:1.

Hakala, T.K., Liitiä, T., Suurnäkki, A., 2013. Carbohydrate Polymers. pp. 102-108.

Hua, X., Zhao, H., Yang, R., Zhang, W., Zhao, W., 2010. J. Food Eng. 100, 302-309.

Kaprelyants, L., Zhurlova, O., Shpyrko, T., Pozhitkova, L., 2017. Food Sci. Technol. 11, 25-34.

Kim, Y.-K., Shin, C., 2018. Curr. Neuropharmacol. 16, 559-573.

Kowalski, K., Mulak, A., 2019. J. Neurogastroenterol. Motil.

Kumar, V., Satyanarayana, T., 2015. Bioresour. Technol. 179, 382-389.

Mafei, T.D.T., Neto, F.S.P.P., Peixoto, G., de Baptista Neto, Á., Monti, R., Masarin, F., 2019. Appl. Biochem. Biotechnol. 190, 197-217.

Mano, M.C.R., Neri-Numa, I.A., da Silva, J.B., Paulino, B.N., Pessoa, M.G., Pastore, G.M., 2018. Appl. Microbiol. Biotechnol.

MarketsandMarkets, 2018. Prebiotic Ingredients Market Worth 7.37 Billion USD by 2023 [WWW Document]. URL https://www.prnewswire.com/news-releases/prebiotic-ingredients-market-worth-737-billion-usd-by-2023-670471503.html (accessed 2.1.20).

Marques, A.I., Serrano, M. de L., Alves, A.M.B., de Sousa, A.P.M., 2019. Cellulose 26, 1977-1992.

Mazlan, N.A., Samad, K.A., Wan Yussof, H., Saufi, S.M., Jahim, J., 2019. Ind. Crops Prod. 129, 575-584.

Monrroy, M., Garcia, J.R., Mendonça, R.T., Baeza, J., Freer, J., 2012. J. Chil. Chem. Soc. 57, 1113-1117.

Moura, P., Barata, R., Carvalheiro, F., Girio, F., Loureiro-Dias, M.C., Esteves, M.P., 2007. LWT - Food Sci. Technol. 40, 963-972.

Moure, A., Gullón, P., Dominguez, H., Parajó, J.C., 2006. Process Biochem. 41, 1913-1923.

Nabarlatz, D., Torras, C., Garcia-Valls, R., Montané, D., 2007. Sep. Purif. Technol. 53, 235-243.

Ohbuchi, T., Sakaino, M., Takahashi, T., Azumi, N., Ishikawa, K., Kawazoe, S., Kobayashi, Y., Kido, Y., 2010. Oral J. Nutr. Sci. Vitaminol. (Tokyo). 54-59.

Ohbuchi, T., Takahashi, T., Azumi, N., Sakaino, M., 2009. Biosci. Biotechnol. Biochem. 73, 2070-2076.

Patil, N. V., Janssen, A.E.M., Boom, R.M., 2014. Chem. Eng. Sci. 106, 86-98.

Qing, Q., Li, H., Kumar, R., Wyman, C.E., 2013. Aqueous Pretreatment of Plant Biomass for Biological and Chemical Conversion to Fuels and Chemicals. pp. 391-415.

Quiñones, T.S., Retter, A., Hobbs, P.J., Budde, J., Heiermann, M., Plöchl, M., Ravella, S.R., 2015. Biofuels 6, 147-155.

Rajagopalan, G., Shanmugavelu, K., Yang, K.L., 2017. Carbohydr. Polym. 167, 158-166.

Roberfroid, M., Gibson, G.R., Hoyles, L., McCartney, A.L., Rastall, R., Rowland, I., Wolvers, D., Watzl, B., Szajewska, H., Stahl, B., Guarner, F., Respondek, F., Whelan, K., Coxam, V., Davicco, M.J., Léotoing, L., Wittrant, Y., Delzenne, N.M., Cani, P.D., Neyrinck, A.M., Meheust, A., 2010. Br. J. Nutr. 104, S1-63.

Samanta, A.K., Jayapal, N., Jayaram, C., Roy, S., Kolte, A.P., Senani, S., Sridhar, M., 2015. Bioact. Carbohydrates Diet. Fibre 5, 62-71.

Selvendran, R.R., March, J.F., Ring, S.G., 1979. Anal. Biochem. 96, 282-292.

Shatalov, A.A., Evtuguin, D. V., Pascoal Neto, C., 1999. Carbohydr. Res. 320, 93-99.

Shortt, C., Hasselwander, O., Meynier, A., Nauta, A., Fernández, E.N., Putz, P., Rowland, I., Swann, J., Türk, J., Vermeiren, J., Antoine, J.M., 2018. Eur. J. Nutr. 57, 25-49.

Silva, F.F., Alves, A.M.B., de Lurdes Serrano, M., de Sousa, A.P.M., 2017. Sep. Purif. Technol. 173, 233-239.

Silveira, M.H.L., Chandel, A.K., Vanelli, B.A., Sacilotto, K.S., Cardoso, E.B., 2018. Bioresour. Technol. Reports 3, 138-146.

Singh, R.D., Banerjee, J., Sasmal, S., Muir, J., Arora, A., 2018. Bioresour. Technol. 256, 110-117.

Swennen, K., Courtin, C.M., Van Der Bruggen, B., Vandecasteele, C., Delcour, J.A., 2005. Carbohydr. Polym. 62, 283-292.

Taha, M., Foda, M., Shahsavari, E., Aburto-Medina, A., Adetutu, E., Ball, A., 2016. Curr. Opin. Biotechnol. 38, 190-197.

TAPPI T 412 om-11, 2011. Moisture in Pulp, Paper and Paperboard. TAPPI Press.

TAPPI T211 om-02, 2002. T211 om-02. Ash in wood, pulp, paper and paperboard: combustion at 525oC. TAPPI test methods.

Vegas, R., Moure, A., Dominguez, H., Parajó, J.C., Álvarez, J.R., Luque, S., 2006. Desalination 199, 541-543.

Wang, T., Li, C., Song, M., Fan, R., 2019. Grain Oil Sci. Technol. 2, 73-77.

Wang, Y., Cao, X., Zhang, R., Xiao, L., Yuan, T., Shi, Q., Sun, R., 2018. RSC Adv. 8, 35211-35217.

Wen, P., Zhang, T., Wang, J., Lian, Z., Zhang, J., 2019. Biotechnol. Biofuels 12, 87.

Yuan, Q.P., Zhang, H., Qian, Z.M., Yang, X.J., 2004. J. Chem. Technol. Biotechnol. 79, 1073-1079.

Zhang, H., Zhou, X., Xu, Y., Yu, S., 2017. J. Wood Chem. Technol. 37, 1-9.

Zhao, H., Hua, X., Yang, R., Zhao, L., Zhao, W., Zhang, Z., 2012. Int. J. Food Sci. Technol. 47, 32-39.

Zhou, X., Xu, Y., 2019. Biofuels 12:272.

**Claims**

1. process for the production of xylooligosaccharides **characterised by** comprising the following steps:

   - concentrating an alkaline filtrate from a Kraft pulp of *Eucalyptus globulus* by ultrafiltration;
   - acid hydrolysis of the resulting alkaline filtrate with nitric acid, at pHs between 0.5 and 1.5 and at temperatures between 70 and 100 ° C;
   - purificating the xylooligosaccharides solution.

2. The process according to claim 1 **characterised in that** the acid hydrolysis takes place at pHs of 0.5 to 1 and at temperatures between 85 and 100 ° C.

3. The process according to claim 1 **characterised in that** the acid hydrolysis takes place at a pH of 0.5 and a temperature of 100 ° C.

4. The process according to any one of the preceding claims **characterised in that** the purification step comprises a pre-purification by nanofiltration followed by solvent precipitation.

**Patentansprüche**

1. Prozess zur produktion von Xylooligosacchariden, **dadurch gekennzeichnet, dass** es die folgenden Schritte umfasst:

- Konzentrieren eines alkalischen Filtrats aus einem Kraftzellstoff von Eucalyptus globulus durch Ultrafiltration;
- saure Hydrolyse des resultierenden alkalischen Filtrats mit Salpetersäure bei pH-Werten zwischen 0,5 und 1,5 und bei Temperaturen zwischen 70 und 100°C;
- Reinigung der Lösung der Xylooligosaccharide.

2. Prozess nach Anspruch 1, **dadurch gekennzeichnet, dass** die saure Hydrolyse bei pH-Werten von 0,5 bis 1 und bei Temperaturen zwischen 85 und 100 °C erfolgt.

3. Prozess nach Anspruch 1, **dadurch gekennzeichnet, dass** die saure Hydrolyse bei einem pH-Wert von 0,5 und einer Temperatur von 100 °C erfolgt.

4. Prozess nach einem der vorherigen Ansprüche, **dadurch gekennzeichnet, dass** der Reinigungsschritt eine Vorreinigung durch Nanofiltration gefolgt von einer Lösungsmittelfällung umfasst.


**Revendications**

1. Procédé de production de xylooligosaccharides **caractérisé en ce qu'**il comprend les étapes de:

- concentration d'un filtrat alcalin provenant d'une pâte Kraft d'Eucalyptus globulus par ultrafiltration ;
- hydrolyse acide du filtrat alcalin obtenu avec de l'acide nitrique, à des pH compris entre 0,5 et 1,5 et à des températures comprises entre 70 et 100 °C ;
- purification de la solution de xylooligosaccharides.

2. Procédé selon la revendication 1 **caractérisé en ce que** l'hydrolyse acide a lieu à des pH de 0,5 à 1 et à des températures comprises entre 85 et 100°C.

3. Procédé selon la revendication 1 **caractérisé en ce que** l'hydrolyse acide a lieu à un pH de 0,5 et à une température de 100°C.

4. Procédé selon l'une quelconque des revendications précédentes **caractérisé en ce que** l'étape de purification comprend une pré-purification par nanofiltration suivie d'une précipitation au solvant.

Fig. 1

Fig. 2

Fig. 3

Fig. 4

Fig. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20130296545 A1
- ES 2346124 A1
- EP 0988402 A1
- CN 102277761 B
- EP 2748341 B1

### Non-patent literature cited in the description

- **AACHARY, A.A. ; PRAPULLA, S.G.** *Compr. Rev. Food Sci. Food Saf.,* 2011, vol. 10, 2-16
- **AKPINAR, O. ; ERDOGAN, K. ; BAKIR, U. ; YILMAZ, L.** *Food Sci. Technol.,* 2010, vol. 43, 119-125
- **AMORIM, C. ; SILVÉRIO, S.C. ; PRATHER, K.L.J. ; RODRIGUES, L.R.** *Biotechnol. Adv.,* 2019, vol. 37
- **APPLETON, J.** *Integr. Med.,* 2018, vol. 17, 28-32
- **BATISTA, G. ; SOUZA, R.B.A. ; PRATTO, B. ; DOS SANTOS-ROCHA, M.S.R. ; CRUZ, A.J.G.** *Bioresour. Technol.,* 2019, vol. 275, 321-327
- **BANERJEE, S. ; PATTI, A.F. ; VIJAYARAGHAVAN, R. ; ARORA, A.** *FOOD BIOPROD PROCESS,* 2019, vol. 117, 38-50
- **BHATIA, R. ; WINTERS, A. ; BRYANT, D.N. ; BOSCH, M. ; CLIFTON-BROWN, J. ; LEAK, D. ; GALLAGHER, J.** *Bioresour. Technol.,* 2020, vol. 296
- **BINNS, N.** Probiotics, prebiotics and the gut microbiota. *International life sciences institute europe concise monograph series,* 2013
- **CAO, L. ; CHEN, H. ; TSANG, D.C.W. ; LUO, G. ; HAO, S. ; ZHANG, S. ; CHEN, J.** *J. Clean. Prod.,* 2018, vol. 178, 572-579
- **CARLSON, J.L. ; ERICKSON, J.M. ; LLOYD, B.B. ; SLAVIN, J.L.** *Curr. Dev. Nutr.,* 2018, vol. 2, 1-8
- **CARVALHO, A.F.A. ; NETO, P. DE O. ; DA SILVA, D.F. ; PASTORE, G.M.** *Food Res. Int.,* 2013
- **CHEN, X. ; CAO, X. ; SUN, S. ; YUAN, T. ; SHI, Q. ; ZHENG, L. ; SUN, R.** *Ind. Crops Prod.,* 2018, vol. 124, 906-911
- **CÓRDOVA, A. ; ASTUDILLO, C. ; ILLANES, A.** *Membrane Technology.,* 2019, 113-153
- **CRYAN, J.F. ; O'RIORDAN, K.J. ; COWAN, C.S.M. ; SANDHU, K. V et al.** *Physiol. Rev.,* 2019, vol. 99, 1877-2013
- Safety of xylooligosaccharides (XOS) as a novel food pursuant to Regulation (EU) 2015/2283. *EFSA J.,* 2018
- **ENAM, F. ; MANSELL, T.J.** *J. Ind. Microbiol. Biotechnol.,* 2019, vol. 46, 1445-1459
- **EVTUGUIN, D.V. ; PASCOAL NETO, C.** Recent advances in Eucalyptus wood chemistry: structural features through the prism of technological response, 2007
- **GIBSON, G.R. ; HUTKINS, R. ; SANDERS, M.E. ; PRESCOTT, S.L. ; REIMER, R.A. ; SALMINEN, S.J. ; SCOTT, K. ; STANTON, C. ; SWANSON, K.S. ; CANI, P.D.** *Nat. Rev. Gastroenterol. Hepatol.,* 2017, vol. 14, 491
- **HACHEM, K. ; FAUGERON-GIRARD, C. ; KAID-HARCHE, M. ; GLOAGUEN, V.** *Cogent Chem.,* 2017, vol. 3, 1
- **HAKALA, T.K. ; LIITIÄ, T. ; SUURNÄKKI, A.** *Carbohydrate Polymers.,* 2013, 102-108
- **HUA, X. ; ZHAO, H. ; YANG, R. ; ZHANG, W. ; ZHAO, W.** *J. Food Eng.,* 2010, vol. 100, 302-309
- **KAPRELYANTS, L. ; ZHURLOVA, O. ; SHPYRKO, T. ; POZHITKOVA, L.** *Food Sci. Technol.,* 2017, vol. 11, 25-34
- **KIM, Y.-K. ; SHIN, C.** *Curr. Neuropharmacol.,* 2018, vol. 16, 559-573
- **KOWALSKI, K. ; MULAK, A.** *J. Neurogastroenterol. Motil.,* 2019
- **KUMAR, V. ; SATYANARAYANA, T.** *Bioresour. Technol.,* 2015, vol. 179, 382-389
- **MAFEI, T.D.T. ; NETO, F.S.P.P. ; PEIXOTO, G. ; DE BAPTISTA NETO, Á. ; MONTI, R. ; MASARIN, F.** *Appl. Biochem. Biotechnol.,* 2019, vol. 190, 197-217
- **MANO, M.C.R. ; NERI-NUMA, I.A. ; DA SILVA, J.B. ; PAULINO, B.N. ; PESSOA, M.G. ; PASTORE, G.M.** *Appl. Microbiol. Biotechnol.,* 2018
- *Prebiotic Ingredients Market Worth 7.37 Billion USD by 2023,* 2018, https://www.prnewswire.com/news-releases/prebiotic-ingredients-market-worth-737-billion-usd-by-2023-670471503.html

- **MARQUES, A.I. ; SERRANO, M. DE L. ; ALVES, A.M.B. ; DE SOUSA, A.P.M.** *Cellulose,* 2019, vol. 26, 1977-1992
- **MAZLAN, N.A. ; SAMAD, K.A. ; WAN YUSSOF, H. ; SAUFI, S.M. ; JAHIM, J.** *Ind. Crops Prod.,* 2019, vol. 129, 575-584
- **MONRROY, M. ; GARCIA, J.R. ; MENDONÇA, R.T. ; BAEZA, J. ; FREER, J.** *J. Chil. Chem. Soc.,* 2012, vol. 57, 1113-1117
- **MOURA, P. ; BARATA, R. ; CARVALHEIRO, F. ; GIRIO, F. ; LOUREIRO-DIAS, M.C. ; ESTEVES, M.P.** *LWT - Food Sci. Technol.,* 2007, vol. 40, 963-972
- **MOURE, A. ; GULLÓN, P. ; DOMINGUEZ, H. ; PARAJÓ, J.C.** *Process Biochem.,* 2006, vol. 41, 1913-1923
- **NABARLATZ, D. ; TORRAS, C. ; GARCIA-VALLS, R. ; MONTANÉ, D.** *Sep. Purif. Technol.,* 2007, vol. 53, 235-243
- **OHBUCHI, T. ; SAKAINO, M. ; TAKAHASHI, T. ; AZUMI, N. ; ISHIKAWA, K. ; KAWAZOE, S. ; KOBAYASHI, Y. ; KIDO, Y.** *Oral J. Nutr. Sci. Vitaminol. (Tokyo),* 2010, 54-59
- **OHBUCHI, T. ; TAKAHASHI, T. ; AZUMI, N. ; SAKAINO, M.** *Biosci. Biotechnol. Biochem.,* 2009, vol. 73, 2070-2076
- **PATIL, N. V. ; JANSSEN, A.E.M. ; BOOM, R.M.** *Chem. Eng. Sci.,* 2014, vol. 106, 86-98
- **QING, Q. ; LI, H. ; KUMAR, R. ; WYMAN, C.E.** *Aqueous Pretreatment of Plant Biomass for Biological and Chemical Conversion to Fuels and Chemicals,* 2013, 391-415
- **QUIÑONES, T.S. ; RETTER, A. ; HOBBS, P.J. ; BUDDE, J. ; HEIERMANN, M. ; PLÖCHL, M. ; RAVELLA, S.R.** *Biofuels,* 2015, vol. 6, 147-155
- **RAJAGOPALAN, G. ; SHANMUGAVELU, K. ; YANG, K.L.** *Carbohydr. Polym.,* 2017, vol. 167, 158-166
- **ROBERFROID, M. ; GIBSON, G.R. ; HOYLES, L. ; MCCARTNEY, A.L. ; RASTALL, R. ; ROWLAND, I. ; WOLVERS, D. ; WATZL, B. ; SZAJEWSKA, H. ; STAHL, B.** *Br. J. Nutr.,* 2010, vol. 104, 1-63
- **SAMANTA, A.K. ; JAYAPAL, N. ; JAYARAM, C. ; ROY, S. ; KOLTE, A.P. ; SENANI, S. ; SRIDHAR, M.** *Bioact. Carbohydrates Diet. Fibre,* 2015, vol. 5, 62-71
- **SELVENDRAN, R.R. ; MARCH, J.F. ; RING, S.G.** *Anal. Biochem.,* 1979, vol. 96, 282-292
- **SHATALOV, A.A. ; EVTUGUIN, D. V. ; PASCOAL NETO, C.** *Carbohydr. Res.,* 1999, vol. 320, 93-99
- **SHORTT, C. ; HASSELWANDER, O. ; MEYNIER, A. ; NAUTA, A. ; FERNÁNDEZ, E.N. ; PUTZ, P. ; ROWLAND, I. ; SWANN, J. ; TÜRK, J. ; VERMEIREN, J.** *Eur. J. Nutr.,* 2018, vol. 57, 25-49
- **SILVA, F.F. ; ALVES, A.M.B. ; DE LURDES SERRANO, M. ; DE SOUSA, A.P.M.** *Sep. Purif. Technol.,* 2017, vol. 173, 233-239
- **SILVEIRA, M.H.L. ; CHANDEL, A.K. ; VANELLI, B.A. ; SACILOTTO, K.S. ; CARDOSO, E.B.** *Bioresour. Technol. Reports,* 2018, vol. 3, 138-146
- **SINGH, R.D. ; BANERJEE, J. ; SASMAL, S. ; MUIR, J. ; ARORA, A.** *Bioresour. Technol.,* 2018, vol. 256, 110-117
- **SWENNEN, K. ; COURTIN, C.M. ; VAN DER BRUGGEN, B. ; VANDECASTEELE, C. ; DELCOUR, J.A.** *Carbohydr. Polym.,* 2005, vol. 62, 283-292
- **TAHA, M. ; FODA, M. ; SHAHSAVARI, E. ; ABURTO-MEDINA, A. ; ADETUTU, E. ; BALL, A.** *Curr. Opin. Biotechnol.,* 2016, vol. 38, 190-197
- Moisture in Pulp, Paper and Paperboard. TAPPI T 412 om-11. TAPPI Press, 2011
- TAPPI T211 om-02, 2002. T211 om-02. Ash in wood, pulp, paper and paperboard: combustion at 525oC. *TAPPI test methods,* 2002
- **VEGAS, R. ; MOURE, A. ; DOMINGUEZ, H. ; PARAJÓ, J.C. ; ÁLVAREZ, J.R. ; LUQUE, S.** *Desalination,* 2006, vol. 199, 541-543
- **WANG, T. ; LI, C. ; SONG, M. ; FAN, R.** *Grain Oil Sci. Technol.,* 2019, vol. 2, 73-77
- **WANG, Y. ; CAO, X. ; ZHANG, R. ; XIAO, L. ; YUAN, T. ; SHI, Q. ; SUN, R.** *RSC Adv.,* 2018, vol. 8, 35211-35217
- **WEN, P. ; ZHANG, T. ; WANG, J. ; LIAN, Z. ; ZHANG, J.** *Biotechnol. Biofuels,* 2019, vol. 12, 87
- **YUAN, Q.P. ; ZHANG, H. ; QIAN, Z.M. ; YANG, X.J.** *J. Chem. Technol. Biotechnol.,* 2004, vol. 79, 1073-1079
- **ZHANG, H. ; ZHOU, X. ; XU, Y. ; YU, S.** *J. Wood Chem. Technol.,* 2017, vol. 37, 1-9
- **ZHAO, H. ; HUA, X. ; YANG, R. ; ZHAO, L. ; ZHAO, W. ; ZHANG, Z.** *Int. J. Food Sci. Technol.,* 2012, vol. 47, 32-39
- **ZHOU, X. ; XU, Y.** *Biofuels,* 2019, vol. 12, 272